# EUROPEAN PATENT SPECIFICATION

(11) **EP 2 951 292 B1**
(45) Date of publication and mention of the grant of the patent: **26.05.2021**
(21) Application number: 14717082.3
(22) Date of filing: 30.01.2014
(51) Int. Cl.: C12N 5/10, C12N 15/87, A61K 48/00

(54) **PLATELETS TRANSFECTED BY EXOGENOUS GENETIC MATERIAL AND PLATELET MICROPARTICLES OBTAINED BY SAID TRANSFECTED PLATELETS, METHOD FOR THE PREPARATION AND USES THEREOF**
DURCH EIN EXOGENES GENETISCHES MATERIAL TRANSFEKTIERTE BLUTPLÄTTCHEN UND AUS DIESEN TRANSFEKTIERTEN BLUTPLÄTTCHEN GEWONNENE BLUTPLÄTTCHEN-MIKROPARTIKEL, VERFAHREN ZUR HERSTELLUNG UND VERWENDUNGEN DAVON
PLAQUETTES TRANSFECTÉES PAR UN MATÉRIAU GÉNÉTIQUE EXOGÈNE ET MICROPARTICULES PLAQUETTAIRES OBTENUES À L'AIDE DES PLAQUETTES TRANSFECTÉES, PROCÉDÉ POUR LEUR PRÉPARATION ET LEURS UTILISATIONS

(30) Priority: 30.01.2013 IT RM20130054
(43) Date of publication of application: 09.12.2015
(73) Proprietor: Plasfer S.r.l., 06121 Perugia (IT)
(72) Inventor: Gresele, Paolo, 06132 Perugia (PG) (IT); Malvestiti, Marco, 06034 Foligno (PG) (IT)
(74) Representative: Minoja, Fabrizio
(86) International application number: PCT/IT2014/000028
(87) International publication number: WO 2014/118817

(56) References cited:
- WO-A1-00/27795
- OHMORI T ET AL.: "Efficient expression of a transgene in platelets using simian immunodeficiency virus-based vector harboding glycoprotein Ibalpha promoter: in vivo model for platelet-targeting gene therapy", FASEB J, vol. 20, 2006, pages E769-E779, XP002695896,
- FUJIMOTO T.T. ET AL: "Production of functional platelets by differentiated embryonic stem (ES) cells in vitro", BLOOD, vol. 102, no. 12, 2003, pages 4044-4051, XP002695897,
- HONG W. ET AL: "Transfection of human platelets with short interfering RNA", CLIN. TRANS. SCI., vol. 4, 2011, pages 180-182, XP002695898, cited in the application
- KONDKAR ALTAF A ET AL: "Transfection of Human Platelets Down-Regulates Endogenous mRNA", , 22 November 2009 (2009-11-22), XP002695899, 51ST ANNUAL MEETING OF THE AMERICAN-SOCIETY-OF-HEMATOLOGY, USA Retrieved from the Internet: URL:https://ash.confex.com/ash/2009/webpro gram/Paper22119.html [retrieved on 5014-07-25]
- Anonymous: "Polyethylenimine", Wikipedia, the free encyclopedia , XP002727771, Retrieved from the Internet: URL:http://en.wikipedia.org/wiki/Polyethyl enimine [retrieved on 2014-07-24]
- Platelets And Thrombopoiesis ET AL: "Regular Article", , 1 January 2013 (2013-01-01), XP055637584, DOI: 10.1182/blood-2012-10- Retrieved from the Internet: URL:https://ashpublications.org/blood/arti cle-pdf/121/19/3908/1218626/3908.pdf [retrieved on 2019-10-30]
- HAEMOSTASEOLOGIE, vol. 34, 2014, pages 301-309,
- TRANSFUSION, vol. 51, 2011, pages 1695-1765,
- BLOOD, vol. 121, 2013, pages 3319-3324,
- BLOOD, vol. 37, 1971, pages 395-412,
- BMC BIOTECHNOLOGY, vol. 10, 2010, pages 1-9,
- BLOOD CELLS, MOLECULES AND DISEASES, vol. 35, 2005, pages 70-73,
- HAEMATOLOGICA, vol. 96, 2011, pages 355-359,
- BLOOD, vol. 119, 2012, pages 1634-1642,
- ARTIFICIAL ORGANS, vol. 35, 2010, pages 137-144,
- Megakaryocyte Development and Platelet Production, Italiano. In Platelets in Thrombotic and Non-Thrombotic Disorders, 2017
- FRONTIERS IN PHARMACOLOGY, vol. 7, 2016, pages 1-11,
- European Medicines Agency, publication of 31-01-2019
- EUROPEAN JOURNAL OF CARDIOVASCULAR NURSING, vol. 1, 2002, pages 273-288,
- NATURE REVIEWS. IMMUNOLOGY, 2019, pages 1-14,

### SEQUENCE LISTING

<110> Gresele, Paolo Malvestiti, Marco
<120> Platelets transfected by exogenous genetic material and platelet microparticles obtained by said transfected platelets, method for the preparation and uses thereof
<130> PCT29419
<150> RM2013000054
   <151> 2013-01-30
<160> 50
<170> PatentIn version 3.5
<210> 1
   <211> 21
   <212> RNA
   <213> Artificial sequence
<220>
   <223> MLL/AFF1 mRNA
<400> 1
   aaaagcagac cuacuccaau g 21
<210> 2
   <211> 23
   <212> RNA
   <213> Artificial sequence
<220>
   <223> siRNA against MLL/AFF1 mRNA
<400> 2
   uguuuucguc uggaugaggu uac 23
<210> 3
   <211> 21
   <212> RNA
   <213> Artificial sequence
<220>
   <223> MLL/AFF1 mRNA
<400> 3
   acuuuaagca gaccuacucc a 21
<210> 4
   <211> 23
   <212> RNA
   <213> Artificial Sequence
<220>
   <223> siRNA against MLL/AFF1 mRNA
<400> 4
   ccugaaauuc gucuggauga ggu 23
<210> 5
   <211> 21
   <212> RNA
   <213> Artificial Sequence
<220>
   <223> MLL/AFF1 mRNA
<400> 5
   aagaaaagca gaccuacucc a 21
<210> 6
   <211> 23
   <212> RNA
   <213> Artificial Sequence
<220>
   <223> siRNA against MLL/AFF1 mRNA
<400> 6
   uuuucuuuuc gucuggauga ggu 23
<210> 7
   <211> 21
   <212> DNA
   <213> Artificial Sequence
<220>
   <223> NPM1/ALK mRNA
<400> 7
   cacuuaguag uguaccgcct t 21
<210> 8
   <211> 21
   <212> DNA
   <213> Artificial Sequence
<220>
   <223> siRNa against NPM1/ALK mRNA
<400> 8
   ttgugaauca ucacauggcg g 21
<210> 9
   <211> 21
   <212> RNA
   <213> Artificial Sequence
<220>
   <223> NPM1/ALK mRNA
<400> 9
   cagcacuuag uaguguaccg c 21
<210> 10
   <211> 21
   <212> RNA
   <213> Artificial Sequence
<220>
   <223> si RNA against NPM1/ALK mRNA
<400> 10
   cugucgugaa ucaucacaug g 21
<210> 11
   <211> 21
   <212> RNA
   <213> Artificial Sequence
<220>
   <223> NPM1/ALK mRNA
<400> 11
   cuuaguagug uaccgccgcu u 21
<210> 12
   <211> 21
   <212> RNA
   <213> Artificial sequence
<220>
   <223> si RNA against NPM1/ALK mRNA
<400> 12
   uugaaucauc acauggcggc g 21
<210> 13
   <211> 21
   <212> RNA
   <213> Artificial Sequence
<220>
   <223> RUNX1/RUNX1T1 mRNA
<400> 13
   ccucgaaauc guacugagaa g 21
<210> 14
   <211> 21
   <212> RNA
   <213> Artificial Sequence
<220>
   <223> si RNA against RUNX1/RUNX1T1 mRNA
<400> 14
   uuggagcuuu agcaugacuc u 21
<210> 15
   <211> 21
   <212> RNA
   <213> Artificial sequence
<220>
   <223> BRAF E599V mRNA
<400> 15
   gcuacagaga aaucucgauu u 21
<210> 16
   <211> 21
   <212> RNA
   <213> Artificial sequence
<220>
   <223> siRNA against BRAF E599V mRNA
<400> 16
   uucgaugucu cuuuagagcu a 21
<210> 17
   <211> 22
   <212> RNA
   <213> Artificial Sequence
<220>
   <223> HRAS G12V mRNA
<400> 17
   gggcgccguc ggucugggca ag 22
<210> 18
   <211> 24
   <212> RNA
   <213> Artificial Sequence
<220>
   <223> siRNA against HRAS G12V mRNA
<400> 18
   uucccgcggc agccacaccc guuc 24
<210> 19
   <211> 21
   <212> RNA
   <213> Artificial Sequence
<220>
   <223> KRAS G12C mRNA
<400> 19
   guuggagcuu gugccguagu u 21
<210> 20
   <211> 21
   <212> RNA
   <213> Artificial Sequence
<220>
   <223> siRNA against KRAS G12c mRNA
<400> 20
   uucaaccucg aacaccgcau c 21
<210> 21
   <211> 21
   <212> RNA
   <213> Artificial Sequence
<220>
   <223> KRAS G12N mRNA
<400> 21
   guuggagcug auggcguagu u 21
<210> 22
   <211> 21
   <212> RNA
   <213> Artificial Sequence
<220>
   <223> siRNA against KRAS G12N mRNA
<400> 22
   uucaaccucg acuaccgcau c 21
<210> 23
   <211> 21
   <212> RNA
   <213> Artificial Sequence
<220>
   <223> KRAS G12V mRNA
<400> 23
   guuggagcug uuggcguagu u 21
<210> 24
   <211> 21
   <212> RNA
   <213> Artificial sequence
<220>
   <223> siRNA against KRAS G12V mRNA
<400> 24
   uucaaccucg acaaccgcau g 21
<210> 25
   <211> 23
   <212> DNA
   <213> Artificial sequence
<220>
   <223> NRAS Q61R mRNA
<400> 25
   cgagaagagu acagugccau gtt 23
<210> 26
   <211> 22
   <212> DNA
   <213> Artificial sequence
<220>
   <223> siRNA against NRAS Q61R mRNA
<400> 26
   ttgcucuucu caugucacgg ua 22
<210> 27
   <211> 21
   <212> DNA
   <213> Artificial sequence
<220>
   <223> TP53 R248W mRNA
<400> 27
   gcaugaacug gaggcccaut t 21
<210> 28
   <211> 21
   <212> DNA
   <213> Artificial sequence
<220>
   <223> siRNA against TP53 R248W mRNA
<400> 28
   ttcguacuug accuccgggu a 21
<210> 29
   <211> 21
   <212> DNA
   <213> Artificial Sequence
<220>
   <223> BCR/ABL1 mRNA
<400> 29
   gcagaguuca aaagcccuut t 21
<210> 30
   <211> 21
   <212> DNA
   <213> Artificial sequence
<220>
   <223> siRNA against BCR/ABL1 mRNA
<400> 30
   ttcgucucaa guuuucggga a 21
<210> 31
   <211> 21
   <212> RNA
   <213> Artificial Sequence
<220>
   <223> BCR/ABL1 mRNA
<400> 31
   cagaguucaa aagccguuca g 21
<210> 32
   <211> 23
   <212> RNA
   <213> Artificial sequence
<220>
   <223> siRNA against BCR/ABL1 mRNA
<400> 32
   ucgucucaag uuuucgggaa guc 23
<210> 33
   <211> 21
   <212> RNA
   <213> Artificial Sequence
<220>
   <223> BCR/ABL1 mRNA
<400> 33
   aauaaggaag aagcccuuca g 21
<210> 34
   <211> 22
   <212> RNA
   <213> Artificial Sequence
<220>
   <223> siRNA against BCR/ABL1 mRNA
<400> 34
   aguuauuccu ucuucgggag uc 22
<210> 35
   <211> 21
   <212> RNA
   <213> Artificial sequence
<220>
   <223> BCR/ABL1 mRNA
<400> 35
   augaauaagg aagaagcccu u 21
<210> 36
   <211> 23
   <212> RNA
   <213> Artificial Sequence
<220>
   <223> siRNA against BCR/ABL1 mRNA
<400> 36
   gguaguuauu cguuguucgg gaa 23
<210> 37
   <211> 21
   <212> RNA
   <213> Artificial Sequence
<220>
   <223> BCR/ABL1 mRNA
<400> 37
   ggagacgcag aagcccuuca g 21
<210> 38
   <211> 23
   <212> RNA
   <213> Artificial Sequence
<220>
   <223> siRNA against BCR/ABL1 mRNA
<400> 38
   uaccucugcg ucuucgggaa guc 23
<210> 39
   <211> 21
   <212> RNA
   <213> Artificial Sequence
<220>
   <223> ETV6/PDGFRB mRNA
<400> 39
   augaagaagc guugcccuuu u 21
<210> 40
   <211> 21
   <212> RNA
   <213> Artificial Sequence
<220>
   <223> siRNA against ETV6/PDGFRB mRNA
<400> 40
   uuuacuucuu cggaacggga a 21
<210> 41
   <211> 21
   <212> DNA
   <213> Artificial Sequence
<220>
   <223> EWSR1/FL11 mRNA
<400> 41
   gcagcagaac ccuucuuaut t 21
<210> 42
   <211> 21
   <212> DNA
   <213> Artificial Sequence
<220>
   <223> siRNA against EWSR1/FL11 mRNA
<400> 42
   ttcgucgucu ugggaagaau a 21
<210> 43
   <211> 21
   <212> DNA
   <213> Artificial Sequence
<220>
   <223> EWSR1/FL11 mRNA
<400> 43
   ggcagcagaa cccuucuuac g 21
<210> 44
   <211> 21
   <212> DNA
   <213> Artificial Sequence
<220>
   <223> siRNA against EWSR1/FL11 mRNA
<400> 44
   qcccqucquc uugggaagaa u 21
<210> 45
   <211> 21
   <212> RNA
   <213> Artificial Sequence
<220>
   <223> EWSR1/FL11 mRNA
<400> 45
   gcagaacccu ucuuaugacu u 21
<210> 46
   <211> 21
   <212> RNA
   <213> Artificial Sequence
<220>
   <223> siRNA against EWSR1/FL11 mRNA
<400> 46
   uucgucuugg ucagaauacu g 21
<210> 47
   <211> 20
   <212> DNA
   <213> Artificial Sequence
<220>
   <223> HPRT forward primer
<400> 47
   tgaggatttg gaaagggtgt 20
<210> 48
   <211> 20
   <212> DNA
   <213> Artificial Sequence
<220>
   <223> HPRT reverse primer
<400> 48
   tgtaatccag caggtcagca 20
<210> 49
   <211> 24
   <212> DNA
   <213> Artificial Sequence
<220>
   <223> Beta 2 Microglobulin forward primer
<400> 49
   tgactttgtc acagcccaag atag 24
<210> 50
   <211> 23
   <212> DNA
   <213> Artificial Sequence
<220>
   <223> Beta 2 Microglobulin reverse primer
<400> 50
   ctctaagttg ccacccctcc tag 23

## Description

The present invention relates to platelets transfected with exogenous genetic material and microparticles deriving from said transfected platelets, a method for their preparation and uses thereof.

More in particular, the invention concerns mature platelets transfected with exogenous genetic material and microparticles deriving from said transfected mature platelets having a high percentage of transfection and able to transport the exogenous genetic material and to transfect acceptor cells with such genetic material and then used for example in gene and cell therapy. The invention further concerns a method for the preparation of mature platelets transfected with exogenous genetic material and microparticles deriving from said mature transfected platelets which permits to obtain high percentages of transfection.

MicroRNAs and their corresponding synthetic siRNA, are small noncoding RNAs which represent an important gene expression regulation mechanism at the post-transcriptional level by the translational repression or induction of specific mRNA decay.

It is known that therapies based on siRNAs always require more effective methods for their transportation in the bloodstream (Yoo et al, Bio-inspired, bioengineered and biomimetic drug delivery carriers, Nature Review, Vol 10; 2011*).* For this purpose, methods which use bacteria, viruses, artificial synthetic structures (micelles, microsomes, etc) and even human cells (erythrocytes, macrophages, lymphocytes and stem cells) as vehicles were developed.

Some clinical trials focused on siRNA delivery *in vivo* from cells harvested from patient, modified with siRNA and then reimplanted or reinfused; this method has been used for example for shRNA delivery transported by lentivirus for the treatment of AIDS, or to increase the immune response against some of the most common types of cancer.

More than 30 clinical trials were carried out in which 21 different types of siRNA or shRNA were used, and particularly focusing on studies aimed to improve siRNA delivery system in order to maximize specificity and, at the same time, minimize toxicity and degradation.

Freedman et al. (Blood 2012 119; 6288-6295) e Sahler J et al (J Thromb Haemost. 2012 Oct 5) studied aspects concerning the transfer of genetic material or proteins from platelet like particles in the first work or from micro-particles produced by megakaryocytes in the second work to other cells (endothelial cells and leukocytes). Said authors do not transfect directly particles or megakaryocytes with the genetic material of interest, but they use a method for the megakaryocytes transfection already disclosed in the art, i.e. of bone marrow platelet precursors. Particularly, in the first article the transfection of megakaryocytes cell line (Meg-01) is carried out by the authors by using "Nucleofector", a device for primary culture transfection by electroporation. In the second article the transfection of megakaryocytes cell line (Meg-01 and Dami) and megakaryocytes collected from the bone marrow of C57BL/6 mice using lentivirus, another well known transfection medium.

The presence of microRNA in human platelets was recently disclosed *(*Landry et al, Existence of a microRNA pathway in anucleate platelets, Nat Struct Mol Biol, 2009; 16(9):961-966*),* but neither the possibility of effectively silencing a gene of interest in platelets introducing a siRNA nor the capability to transfer siRNA from platelets or microparticles deriving from them to other cells using platelets as transporters.
In another work *(*Hong et al. Transfection of human platelet with short interfering RNA; Clin Transl Sci. 2011*),* the attempt to achieve a silencing of platelet mRNA using the common methods of cell transfection was described.

But the obtained results showed low transfection efficacy (less than 9%) so as to not permit to obtain satisfying silencing of mRNAs of interest. Particularly, the above-mentioned article demonstrates that siRNAs inserted into the platelets are capable to silence only partially one mRNA target present in the same platelet.

Therefore, the results demonstrate a silencing that may not be considered of biological relevance, as experimentally demonstrated by the inventors of the present invention: overall, in fact, only about 2% of silencing in the whole platelet population was obtained.

According to the tests performed by the inventors of the present invention on human platelets, the silencing may be considered sufficient only when the transfection efficacy exceeds at least 20%. Generally, a transfection with an efficacy of at least 80% is considered optimal.

In the above-mentioned article, the use of means capable to induce "cationic-lipid mediated delivery", e.g. lipofectamine, used to transfect platelets, is described. A cationic lipid has the characteristic, typical of lipids, to be non-polar/hydrophobic, but it even contains a cation able to confer characteristics of polarity/hydrophilicity. The siRNA penetration through membranes is obtained through the above-described physical and chemical properties of lipofectamine. In view of the above, Hong's observation may exclusively be referred to the transfection obtained through a molecule with that specific physical and chimical characteristics and not to transfection obtained with other molecules, as for example, with a mixture of molecules of different nature with respect to the cationic lipid.

Tests for the in vitro transfection of platelet progenitors transfected with genetic material with high efficacy were previously carried out (Ohmori et al. Efficient expression of a transgene in platelets using simian immunodeficiency virus-based vector harboring glycoprotein lbα promoter: in vivo model for platelet targeting gene therapy; FASEB 20 2006; Fujimoto et al. Production of functional platelets by differentiated embryonic stem cell in vitro; Blood 2003, 102). Particularly, in the article of Fujimoto et al. (Production of functional platelets by differentiated embryonic stem cell in vitro; Blood 2003, 102), platelet-like cytoplasmic particles artificially produced in laboratory starting from hematopoietic stem cells were used, but not human platelets. Therefore, platelet-like particles used in Fujimoto et al. are not functionally, antigenically and morphologically comparable to mature circulating platelets isolated from peripheral blood. Furthermore, according to Fujimoto et al., platelet fragments are produced starting from embryonic stem cells, i.e. cells which are difficult to find for a possible clinical use in patients, and the number of platelets obtained with the described method would surely not sufficient for a gene therapy. This concept is even clarified in one review dated 2011 (Sun S et al. In Vitro Megakaryocyte Production and Platelet Biogenesis: State of the Art; Transfus Med Rev. 2010 January; 24(1): 33-43) in which the author concludes that, based on the state of the art, it is not possible to use platelet fragments produced in vitro for the in vivo use in human: "However, the number of platelets produced in vitro per MK are orders of magnitude lower than what occurs in vivo and the quality of the platelets generated is unclear. The current inefficiency of this process raises issues of whether cytokines are delivered at the appropriate time, sequence, concentration, in the right combination and/or whether there are cytokines that are yet to be discovered."

The non-identity between blood platelet fragments and mature platelets is even experimentally confirmed in Gandhi et al. (Gandhi et al., A novel strategy for generating platelet-like fragments from megakaryocytic cell lines and human progenitor cells; Blood cell, molecules and disease, 35, 2005) that read: "Although we can isolate PFs (Platelet Fragments) from UT-7/TPO cells with aggregation properties similar to platelets, electron microscopy demonstrates that these fragments do not have the usual structure of normal platelets (i.e., dense granules and circumferential tubulin ring)". Furthermore, the name through this particles are usually indicated, i.e.: "Platelet fragment o platelet like particles", explicitly indicates a non-identity with in vivo circulating mature platelets.

Ohmori et al. (Ohmori et al. Efficient expression of a transgene in platelets using simian immunodeficiency virus-based vector harboring glycoprotein lbα promoter: in vivo model for platelet targeting gene therapy; FASEB 20 2006) suggest that the bone marrow progenitors of platelets, genetically modified, are transplanted in the bone marrow of patients so as to generate genetically modified platelets in vivo. However, at the current state of scientific knowledge, the method described in Ohmori et al. would involve an high risk of clinical complications and high costs related to a stem cell transplant procedure, moreover, it does not permit to obtain a sufficient number of genetically modified circulating platelets. The disadvantages of a method like that one described in Ohomori et al. are explicitly described in Poncz et al. (Poncz M et al. Challenges and promises for the development of donor-independent platelet transfusions; Blood 2013-121/17 3319-3324) in which it is believed that: "In vivo platelet generation from infused megakaryocytes is encouraging and may become an alternative to ex vivo thrombopoiesis, but this approach is limited by the time delay in achieving a maximal rise in platelet counts and the potential concerns with the infusion of whole cells, particularly in patients with actual or potential pulmonary disease".

Furthermore, in the methods described in Ohmori et al. (Efficient expression of a transgene in platelets using simian immunodeficiency virus-based vector harboring glycoprotein lbα promoter: in vivo model for platelet targeting gene therapy; FASEB 20 2006) and Fujimoto et al. (Production of functional platelets by differentiated embryonic stem cell in vitro; Blood 2003, 102), "Platelet like particles" transfection is obtained in an indirect manner. Particularly, cell precursors of platelet-like particles are transfected with the gene material of interest. However, the production of the above-mentioned particles transfected with genetic material is obtained only at a later step, which necessarily determines a lost of the efficacy of the obtained transfection degree.

In both documents cited above, a platelet population in which the transfected platelet percentage do not exceed 20% of the total, as expressly written in Ohmori et al., "In our system, the transduction of hematopoietic stem cells with an SIV lentiviral vector resulted in the expression of the stransgene in ≈20% of platelets" and in Fujimoto et al. "We could obtain as many as 108 platelets in the culture supernatant from 104 ES cells." is obtained. A number of platelets of 10⁸ is several orders of magnitude lower than the number of circulating platelets in the blood and, therefore, it is clearly lower than 20% of total circulating platelets.

Therefore, in view of the above, it is well evident the need to have a new method of transportation and direct transfection of mature platelets with genetic material which overcomes disadvantages of known methods.

The inventors of the present invention found a new method for inserting (transfection) foreign genetic material, such as RNA (siRNA, shRNA, ceRNA) and in general other genetic material, such as, for example plasmids, into human blood platelets. Particularly, a procedure starting from the isolation of human platelets from the peripheral blood of a donor, passing for obtaining resuspended platelets in a resuspension medium, both in the case it is the same plasma of the donor or a resuspension buffer, up to the transfection of the same platelets with a genetic material such as a small interfering RNA (siRNA) directed against a specific target mRNA, is provided. The method of the invention allows platelet transfection also with type of genetic material different from siRNA (e.g. shRNA or plasmids) that may represent further routes in order to obrtain gene silencing or introduce new genetic material so as to improve the efficacy of gene therapies currently used or under study or introducing new ones.

Through the method of the present invention it is possible to obtain platelets or microparticles of platelet origin able to contain a concentration of genetic material sufficient to silence the mRNA of interest within the same platelets or in other cells whereby plateles or microparticles of platelet origin come into contact. The platelets or microparticles of the invention therefore may be advantageously used in the medical field.

The capability of microparticles produced from nucleated cells, e.g. microparticles, microvescicles, exosomes, to transfer genetic material to other "acceptor" cells is well known in literature. The method of the present invention allows to produce microparticles of platelet origin from platelets transfected with genetic material, such as specific siRNA, directly from human platelets. The microparticles may be used as vehicles of genetic material, such as siRNA, in order to silence or manipulate genes of interest in white cell blood, in the endothelium, or in other cells of organs and tissues.

The present invention finds application in several medical fields. The transfection of specific siRNA into platelets permits to interfere, for example, with the mechanisms which regulate the direct partecipation of platelets to ischemic heart disease, inflammation, metastasis, tissue damage or, in transfusion medicine, promoting the hemostatic conservation and efficacy of platelets preventing the so-called Platelet Storage Lesion.

Furthermore, the known capability of platelets to closely interact with other cells, among which the endothelial cells, the smooth muscle cells of the vascular wall, several white blood cell subpopulations and cancer cells, ensure that molecules transfected into platelets are transferred into the above-listed cells. Acceptor cell transfection thus allows to modulate their genetic expression, interfering with pathological conditions in which platelets are not directly involved, such as, several types of cancer, diseases due to eukaryotic and viral pathogens, post-transplant rejection.

The method of the invention allows to obtain the transfection of genetic material selected by the researcher, such as siRNA, in human platelets with high efficacy. The thus obtained silencing of platelet mRNAs is stable and lasts for the entire life-time of transfected platelets. In fact, platelets do not have a nucleus and therefore they are not able to synthesize new mRNA. Therefore transfected platelets may be used to silence genes of pathophysiological interest in human platelets so as to block their involving in several diseases. Particularly, an excellent percentage of transfection using siRNA were obtained (about 95%).

Furthermore the inventors of the present invention demonstrated that siRNAs transfected into platelets are then transferred into microparticles produced by the same platelets. Both microparticles and platelets transfected with genetic material are able to transfer in vivo said material to "acceptor" cells and, particularly, to cells involved in pathologies in which platelets do not have a main role, thus being able to modulate, through platelet microparticles, or platelets, pathologies not primarily deriving from the same platelets.

The fast platelet turn-over in the circulation further allows to minimize siRNA permanence in blood after the release towards target cells, reducing the risk of non-selectivity of the therapeutic intervention. The short times required in order to obtain the transfection of the method of the invention further allow to reinfuse transfected platelets intravenously in the same subject from which they were collected during only once session, thus eliminating the risk of alloimmunization and rejection of transfected platelets.

In fact, the practice used in transfusion medicine of separating platelets and plasma (separately or both) from a donor reinfusing the same with the remaining part of separated blood (corpuscular part), directly in the same transfusion cycle (platelet-plasmapheresis), may permit to collect platelets, transfect them, resuspend them into plasma and reinfusing them.

It is therefore a specific object of the present invention a population of mature platelets, preferably isolated from peripheral blood, transfected with exogenous genetic material and/or platelet microparticles (i.e. platelet fragments) obtained from said mature transfected platelets, wherein the percentage of mature transfected platelets ranges from 20% to 100%, preferably it is higher than 20%, even more preferably it ranges from 50% to 100% and even more preferably from 80% to 100% of the entire platelet population, wherein said platelet microparticles are those containing said exogenous genetic material. The genetic material with which platelets may be transfected may be selected from the group consisting of siRNA, shRNA, ceRNA, DNA, plasmids and in general materials of a genetic nature.

Therefore, according to the present invention, the exogenous genetic material is used to transfect and genetically modify human mature platelets and/or platelet microparticles. Therefore the invention concerns the use of the population of mature platelets transfected with exogenous genetic material and/or platelet microparticles according to the invention as vector in the transportation of genetic material and/or in the transfection of genetic material to acceptor cells.

Furthermore, it is an object of the present invention to provide a population of mature platelets transfected with exogenous genetic material and/or platelet microparticles according to the invention for the use in gene therapy or cell therapy. Particularly, the target gene of the gene therapy may be a messenger RNA present in the same platelets or in the acceptors cells. For example, when said genetic material is selected from the group consisting of siRNA, shRNA, or in said siRNA, shRNA contained in plasmids, the gene therapy consists in the post transcriptional silencing of a target gene that may belong to the same platelets or acceptor cells.

The method of transfection according to the present invention may be used, for example, to insert a siRNA directed against the mRNA of Thrombospondin-1 (TSP-1) within human platelets in order to regulate the amount of the same mRNA in patients with solid tumors (Platelet-derived thrombospondin-1 is a critical negative regulator and potential biomarker of angiogenesis. Blood, 2011).

In fact, this mRNA was observed to play an important role in tumor angiogenesis; the transfer of a siRNA anti mRNA for TSP-1 from platelets, transfected with it, to tumor cells in vivo could represent a selective "chemotherapy", aimed to avoid tumor recurrences after interventions.

The siRNA used in the experimentations as described below are siRNA DICER induced commercially available and sold by numerous companies of the field. Numerous sequences of siRNAs used for clinical trials directed against oncogenes or characteristic sequences of pathogen genomes (HIV-1) are also known. Their effective transfection in platelets therefore represents a therapeutic innovative and effective mean against diseases of the cardiovascular system (atherosclerosis, cardiac hypertrophy and fibrosis, cardiac arrhythmogenesis); neoplasms (solid tumors of different origin, leukemias, myelomas); diseases due to eukaryotic pathogens (mycosis, malaria) or viral (AIDS, viral hepatitis).

Therefore, mature platelets transfected according to the present invention and the microparticles obtained from them may be used in the treatment of the above mentioned diseases.

As for shRNAs or plasmids, uses are similar, clearly with the advantage that silencings with higher duration than that one obtainable with siRNAs may be obtained.

Depending on the type of pathology and above all the genetic characteristics of the disease, siRNAs with different sequences will be introduced within platelets so as to silence mRNAs that, if translated, are fundamental for pathology evolution and development.

In table 1 a series of siRNAs usable against common oncogenes and transfectable in mature platelets according to the present invention (siRNA and miRNA gene silencing-Humana Press) are listed. In table 1, right column, siRNA sequences showing both RNA strands are reported (the higher sequence represents the mRNA and the lower sequence the siRNA directed against the mRNA); in the left column, the names of genes against which the siRNA is directed are shown; in case of "point mutation" also the mutation expressed as single amino acid change is specified. The categories in which the table is divided are referred to the type of oncogene: "point mutation", i.e. the most common point mutations that determine the conversion of that determined proto-oncogene to oncogene, and "fusion gene" where the oncogene is generated by translocation.

**Table 1**

| Oncogene | |
|---|---|
| MLL/AFF1 | AAAAGCAGACCUACUCCAAUG (SEQ ID NO:1) |
| | UGUUUUCGUCUGGAUGAGGUUAC (SEQ ID NO:2) |
| MLUAFF1 | ACUUUAAGCAGACCUACUCCA (SEQ ID NO:3) |
| | CCUGAAAUUCGUCUGGAUGAGGU (SEQ ID NO:4) |
| MLUAFF1 | AAGAAAAGCAGACCUACUCCA (SEQ ID NO:5) |
| | UUUUCUUUUCGUCUGGAUGAGGU(SEQ ID NO:6) |
| NPM1/ALK | CACUUAGUAGUGUACCGCCtt (SEQ ID NO:7) |
| | ttGUGAAUCAUCACAUGGCGG (SEQ ID NO:8) |
| NPM1/ALK | CAGCACUUAGUAGUGUACCGC (SEQ ID NO:9) |
| | CUGUCGUGAAUCAUCACAUGG (SEQ ID NO:10) |
| NPM1/ALK | CUUAGUAGUGUACCGCCGCUU (SEQ ID NO:11) |
| | UUGAAUCAUCACAUGGCGGCG (SEQ ID NO:12) |
| RUNX1/RUNX1T1 | CCUCGAAAUCGUACUGAGAAG (SEQ ID NO:13) |
| Point Mutation | UUGGAGCUUUAGCAUGACUCU (SEQ ID NO:14) |
| BRAF E599V | GCUACAGAGAAAUCUCGAUUU (SEQ ID NO:15) |
| | UUCGAUGUCUCUUUAGAGCUA (SEQ ID NO:16) |
| HRAS G12V | GGGCGCCGUCGGUCUGGGCAAG (SEQ ID NO:17) |
| | UUCCCGCGGCAGCCACACCCGUUC (SEQ ID NO:18) |
| KRAS G12C | GUUGGAGCUUGUGCCGUAGUU (SEQ ID NO:19) |
| | UUCAACCUCGAACACCGCAUC (SEQ ID NO:20) |
| KRAS G12N | GUUGGAGCUGAUGGCGUAGUU (SEQ ID NO:21) |
| | UUCAACCUCGACUACCGCAUC (SEQ ID NO:22) |
| KRAS G12V | GUUGGAGCUGUUGGCGUAGUU (SEQ ID NO:23) |
| | UUCAACCUCGACAACCGCAUG (SEQ ID NO:24) |
| NRAS Q61 R | CGAGAAGAGUACAGUGCCAUGtt (SEQ ID NO:25) |
| | ttGCUCUUCUCAUGUCACGGUA (SEQ ID NO:26) |
| TP53 R248W | GCAUGAACUGGAGGCCCAUtt (SEQ ID NO:27) |
| | ttCGUACUUGACCUCCGGGUA (SEQ ID NO:28) |

| Fusion Gene | |
|---|---|
| BCR/ABL 1 | GCAGAGUUCAAAAGCCCUUtt (SEQ ID NO:29) |
| | ttCGUCUCAAGUUUUCGGGAA (SEQ ID NO:30) |
| BCR/ABL 1 NO:31 ) | CAGAGUUCAAAAGCCGUUCAG (SEQ ID |
| NO:32) | UCGUCUCAAGUUUUCGGGAAGUC (SEQ ID |
| BCR/ABL 1 NO:33) | AAUAAGGAAGAAGCCCUUCAG (SEQ ID |
| NO:34) | AGUUAUUCCUUCUUCGGGAGUC (SEQ ID |
| BCR/ABL 1 NO:35) | AUGAAUAAGGAAGAAGCCCUU (SEQ ID |
| NO:36) | GGUAGUUAUUCGUUGUUCGGGAA (SEQ ID |
| BCR/ABL 1 NO:37) | GGAGACGCAGAAGCCCUUCAG (SEQ ID |
| NO:38) | UACCUCUGCGUCUUCGGGAAGUC (SEQ ID |
| ETV6/PDGFRB NO:39) | AUGAAGAAGCGUUGCCCUUUU (SEQ ID |
| NO:40) | UUUACUUCUUCGGAACGGGAA (SEQ ID |
| EWSR1/FL11 | GCAGCAGAACCCUUCUUAUtt (SEQ ID NO:41) |
| | ttCGUCGUCUUGGGAAGAAUA (SEQ ID NO:42) |
| EWSR1/FL11 | GGCAGCAGAACCCUUCUUAcg (SEQ ID NO:43) |
| | gcCCGUCGUCUUGGGAAGAAU (SEQ ID NO:44) |
| EWSR1/FL11 | GCAGAACCCUUCUUAUGACUU (SEQ ID NO:45) |
| | UUCGUCUUGGUCAGAAUACUG (SEQ ID NO:46) |

Based on the above said, the present invention also relates to platelets transfected with siRNA listed in table 1, microparticles obtained from them and related uses in the treatment of diseases related to the listed genes.

It is a further object of the present invention, a method for platelet transfection with exogenous genetic material, said method comprising or consisting of the following steps:
a) adding a mixture comprising or consisting of genetic material and a transfection medium, said transfection medium comprising ethyl alcohol and at least one polyamine selected from polyethylenimine and polylysine to a suspension of isolated mature platelets suspended in a suitable suspension medium, such as plasma; and
b) incubating until obtaining a percentage of transfected platelets equal at least to 20%, preferably from 50 to 100%, even more preferably from 80 to 100%, of the entire platelet population, interrupting the transfection and isolating the mature transfected platelets. The methods to determine the percentage of transfection are well known to one skilled in the art. For example, citofluorometry may be employed by using fluorescent probes, or PCR real-time. The incubation times may range from 1 minute to 24 hours, generally they range from 1 minute to 3 hours, preferably from 5 minutes to 30 minutes, even more preferably from 5 to 10 minutes.

The present invention further concerns a method for the preparation of platelet microparticles deriving from mature platelets transfected with exogenous genetic material, said method comprising or consisting of the following steps: a) adding a mixture comprising or consisting of genetic material and a transfection medium, said transfection medium comprising ethyl alcohol and at least one polyamine selected from polyethylenimine and polylysine to isolated mature platelets suspendend in a suitable suspension medium, such as plasma; and b) incubating, possibly stimulating platelet activation, until obtaining microparticles, interrupting the transfection and isolating microparticles or a platelet and microparticles mixture. The methods to determine the formation of microparticles are well known to one skilled in the art, for example their formation may be verified by flow-cytometry with standard techniques.

The stimulation of platelet activation has the aim to induce a greater microparticles production and may be performed, for example, by adding thrombin at a final concentration of 1 unit/milliliter or using collagen, TRAP, arachidonic acid and/or other stimuli, well known to one skilled in the art.

As above mentioned, the genetic material is selected from the group consisting of siRNA, shRNA, ceRNA, DNA, plasmids and other materials of genetic nature. It is advisable that the genetic material to be transfected is used at a concentration between 20-200 nM, likewise to what used for any cell transfection.

The transfection is optimally carried out using a mixture comprising or consisting of ethyl alcohol and at least one polyamine selected from polyethylenimine and polylysine. As well as this components, lipids or platelet phospholipid extracts may be added. The transfection efficacy mainly depends on polyamines, whereas platelet phospholipid extracts seem to have a moderate effect enhancing transfection and mitigating the cytotoxic effects of the transfection mixture. The concentration of ethyl alcohol may range from 0.5% to 3.5%, preferably from 2 to 3%, even more preferably 3%. The transfection efficacy, as for alcohol concentration in the medium, is dose-dependent, reaching the maximum efficiency at 3%. The amount of the solution of polylysine or polyethylenimmine diluted with water to 0.1% may range from 1 to 150 µl, preferably from 1 to 140 µl, even more preferably 3 µl.

Lipid concentration may range from 10 to 20 µl, preferably from 12 to 16 µl, even more preferably 15.6 µl.
The optimal transfection was obtained with an amount of 3 µl of a polyethylenimmine solution diluted with water to 0.1% added to the reaction mix and 15.6 microliters of platelet phospholipid extracts (Platelet Extract Reagent).

Therefore, the present invention also concerns a transfection medium of mature platelets with exogenous genetic material, wherein said transfection medium comprises or consists of ethyl alcohol and at least one polyamine selected from polyethylenimine and polylysine, and possibly, lipids or phospholipid extracts.

Using a commercial reagent named Ribojuice (Merck), comprising ethyl alcohol and at least one polyamine, a transfection yield comparable to that one obtained with the transfection medium of the present invention is obtained.

The concentration of mature platelet in suspension of step a) preferably may be higher than 20,000 platelets per microliter and less than 2 millions, preferably from 200,000 to 1,000,000 per microliter, even more preferably 250,000-350,000, even more preferably 300,000 per microliter. Platelet suspension media used during the experimental trials were RPMI 1640 and IMDM and both of them showed to be equally effective.

The incubation times usable for platelet transfection range from 5 minutes to 1 hour; however using short times is preferred (e.g. 5 minutes) in order to reduce cytotoxic effects of the transfection reagent on platelets. Instead, the times for platelet transfection with production of microparticles range from 24 to 48 hours of incubation.

The method according to the present invention ensures a high efficacy of siRNA transfection into human mature platelets and also a high capability of microparticles deriving from platelets and transfected with siRNA of transferring siRNAs of interest to others cells, in vitro and in vivo.

The present invention will be now described, for illustrative but not limitative purposes, according to its preferred embodiments, with particular reference to the figures of the enclosed drawings, wherein:

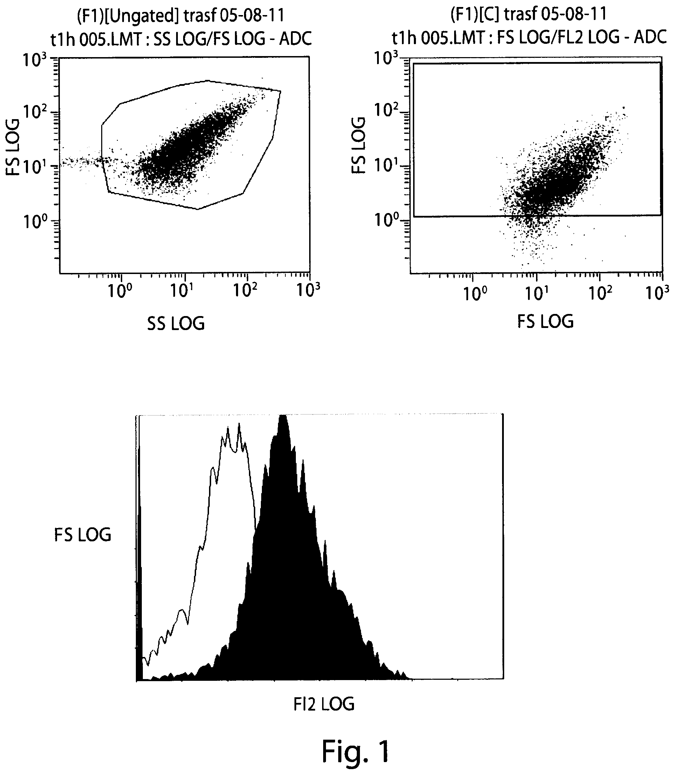

Figure 1 shows: A. Flow-cytometry analysis of the platelet population after transfection obtained by the process described in example 1. The use of scatters on a logarithmic scale allows the view of the platelet population based on morphology. B. Flow-cytometry analysis of the percentage of platelets positive to siRNA marked with fluorescence. C. Flow-cytometry comparison between non-transfected platelets (left curve) with transfected platelets (right curve). The shift towards higher abscissa values corresponding to a greater fluorescence, allows to define the percentage of platelets positive to marked siRNA. FS - forward scatter; SS - side scatter; Fl2 - emission band of the fluorofore of the siRNA.
Figure 2 shows the percentages of platelets positive to fluorescent siRNA in relation to the incubation times with the transfection medium.
Figure 3 shows the expression percentage of mRNA of HPRT1 gene in platelets washed and co-incubated with siRNA against mRNA of HPRT1 measured by semi-quantitative PCR.
Figure 4 shows the comparison semi-quantitative PCR after transfection carried out with siRNA directed against HPRT1 between the target gene and one gene of the control (B2M). In figure A, PCR analysis was carried out amplifying HPRT1 gene, in figure B with B2M (β-2 Microglobulin) gene. L-Ladder; P-washed platelets; P1 washed platelets co-incubated with siRNA; P2 platelets transfected with siRNA.
Figure 5 shows the results of the flow-cytometry analysis of microparticles produced after 24h of incubation of platelets washed with the transfection medium added with fluorescent marked siRNA.
Figure A shows the morphological region where microparticles are comprised previously set with spheres of appropriate sizes (Megamix Beads). I: 0.5 µm; J: 0.9 µm; G: I+J. In figure B the presence of CD61 in the microparticles (on the abscissa-Fl1 Log) in order to show the platelet origin of microparticles and the presence of flurescent marked siRNA (on the ordinate-Fl2) is underlined.

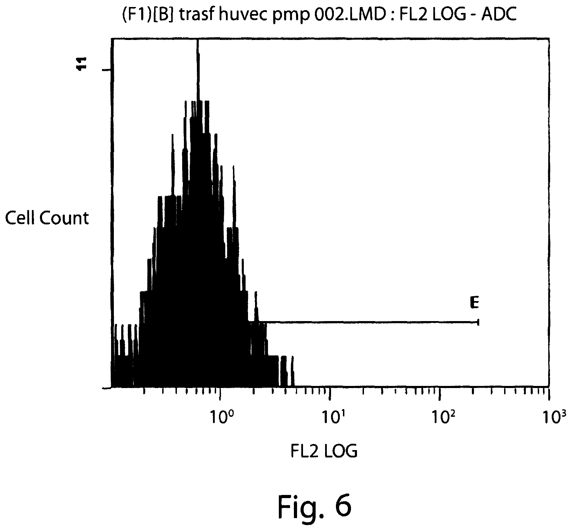

Figure 6 shows the results of the flow-cytometry analysis of endothelial cells (HUVEC) co-incubated with microparticles of platelet origin for 6 hours; the platelet microparticles were prepared starting from a platelet suspension in a transfection medium added with fluorescent marked siRNA, which, after 24 hours, was ultra-centrifuged for 1 hour in order to allow the isolation and the resuspension of microparticles in RPMI 1640. 38% of the endothelial cells results as positive for the siRNA previously transfected into platelets.

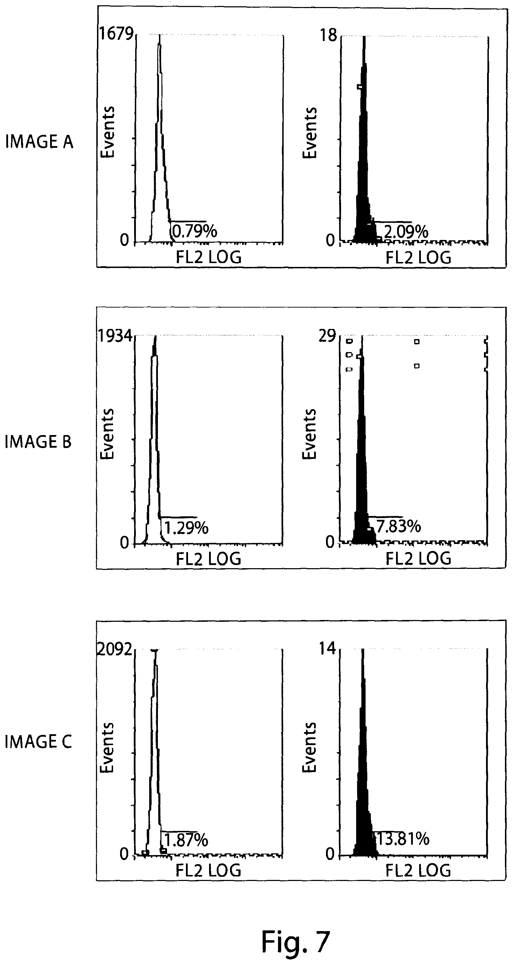

Figure 7 shows the results of the flow-cytometry analysis of murine leukocytes obtained from NOS/SCID mice 24 h after the inoculum of human platelets transfected with fluorescent siRNA. Image A: control leukocytes, the mouse was inoculated with non-transfected human platelets; in the graph on the left the sample was not incubated with antiCD45 antibody; on the right the sample was incubated with antiCD45 antibody; 2.08% of positivity. ImageB: transfected leukocytes, the mouse was inoculated with human platelets transfected with fluorescent siRNA; in the graph on the left the sample was not incubated with antiCD45 antibody; on the right the sample was incubated with antiCD45 antibody; 7.83% of positivity. Image C: leukocytes obtained from mouse after injection in vivo of platelets-activanting agents. The mouse was inoculated with human platelets transfected with fluorescent siRNA and stimulated, one hour after the inoculum, through the injection of 0.1 ml of a solution containing 150 µg/ml of Collagen and 0.9 µg/ml di epinephrine; in the graph on the left the sample was not incubated with antiCD45 antibody; on the right the sample was incubated with antiCD45 antibody; 13.81% of positivity.

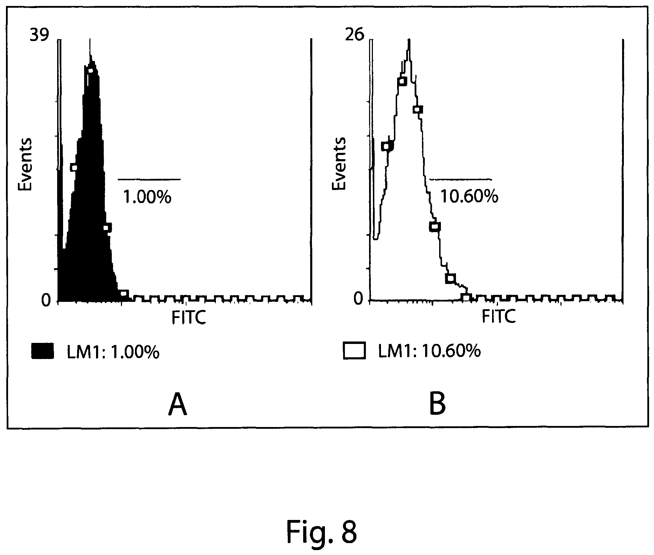

Figure 8 shows: Image A: control carried out with non-transfected platelets; Image B: platelets transfected with Pmax plasmid (Lonza), 10.6% resulted positive and therefore expresses an active GFP.

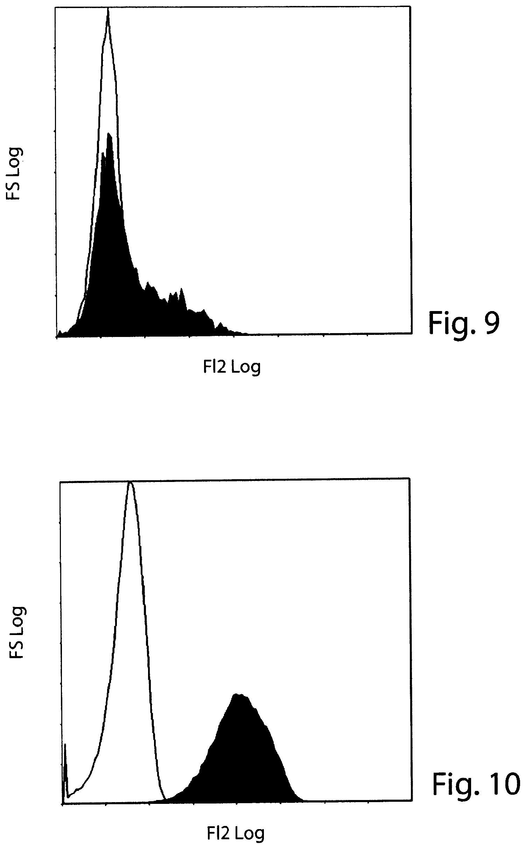

Figure 9 shows the comparison in flow-cytometry between non-transfected platelets (left curve) with transfected platelets (right curve) according to example 4. The shift towards higher abscissa values corresponding to a greater fluorescence, allows to define the percentage of platelets positive to marked siRNA. FS - forward scatter; SS - side scatter; Fl2 - emission band of the fluorofore of the siRNA.
Figure 10 shows the comparison in flow-cytometry between non-transfected platelets (left curve) with transfected platelets (right curve) according to example 5. The shift towards higher abscissa values corresponding to a greater fluorescence, allows to define the percentage of platelets positive to marked siRNA. FS - forward scatter; SS - side scatter; Fl2 - emission band of the fluorofore of the siRNA.

### EXEMPLE 1: Method of transfection of platelets or microparticles and evaluation of the percentage of transfection

### Materials and methods

### Method of transfection of platelets or microparticles

Venous peripheral blood was harvested and collected in tubes containing 3.8% of sodium citrate as anticoagulant (1:9 citrate-blood v/v).
1 The sample was centrifuged at 120 g for 10 minutes in order to obtain PRP (platelet-rich plasma).
2 Proceeding in the isolation of plasma platelets by common methods (platelet washing or gel-filtration).
3 Platelets thus isolated, were resuspended in RPMI 1640 added with antibiotics (100 U of Penicillin and 100 U of Streptomycin) or in the same plasma of the donor, at a concentration comprised between 2x10⁵ and 1x10⁶ per microliter, the lower limit of said concentration being 130,000 platelets/microliter, and rates of one milliliter were transferred to 24-well plate and incubated at 37°C in controlled atmosphere of 5%CO₂.
4 Then, the transfection medium is prepared, inserting 32 microliters of Ribojuice (Merck-Millipore) and 168 microliters of RPMI 1640 into a tube so a to achieve, in both cases, the total amount of 200 microliters.
5 After waiting for 5 minutes, the siRNA of interest was added to the above-mentioned mixture to the maximum concentration of 200 nM and after 15 minutes of incubation at room temperature the solution was transferred into the well in which 1 ml of platelet suspension had previously been placed.
6 After 5 minutes of incubation at 37°C, the transfection was interrupted: by platelet centrifugation (to 3,000 g for 10 minutes in the presence of PGI2 0.2 microM and, then, respuspending them in 3 ml of the culture medium RPMI 1640) or by diluting the transfection medium with the culture medium RPMI 1640 until achieving a total amount of 7 milliliters.
7 Within 2 hours after the interruption of the transfection reaction, it is possible to point out (by PCR or rt-PCR) the degradation of the mRNA of interest, a specific target of the siRNA previously inserted into platelets.

Instead, in order to obtain platelet microparticles containing the siRNA previously transfected into platelets, it is necessary to carry out the previously described operations up to point 5 and then continuing as follows:
1. Incubating platelet suspension at 37°C in controlled atmosphere of 5%CO₂ together with the transfection medium for 24/48 h. Or alternatively stimulating them with 2.5 mM of CaCl₂, 20 µg/ml of Collagen and 1 U/ml of Thrombin for 30 minutes at 37°C and stopping the stimulus with 2.5 mM of EDTA.
2. Centrifuging platelets at 3,000 g for 10 minutes.
3. Recovering supernatant and further centrifuging it at 12,000 g for 10 minutes in order to remove possible remained platelets.
4. Centrifuging supernatant at 160,000 g for 1 hour at 4°C.
5. Removing supernatant and resuspending microparticle pellet in 1 milliliter of RPMI 1640 (verifying microparticles concentration using a flow-cytometer).

The composition of RPMI 1640 medium is (grams/liter): the list of components expressed as grams/liter
L-Arginine 0.2
L-Asparagine 0.05
L-Aspartic Acid 0.02
L-Cystine·2HCl 0.0652
L-Glutamic Acid 0.02
L-Glutamine 0.3
Glycine 0.01
L-Histidine 0.015
Hydroxy-L-Proline 0.02
L-Isoleucine 0.05
L-Leucine 0.05
L-Lysine-HCl 0.04
L-Methionine 0.015
L-Phenylalanine 0.015
L-Proline 0.02
L-Serine 0.03
L-Threonine 0.02
L-Tryptophan 0.005
L-Tyrosine·2Na·2H2O 0.02883
L-Valine 0.02
Biotin 0.0002
Choline Chloride 0.003
Folic Acid 0.001
myo-Inositol 0.035
Niacinamide 0.001
D-Pantothenic Acid Hemicalcium 0.00025
PABA 0.001
Pyridoxine·HCl 0.001
Riboflavin 0.0002
Thiamine·HCl 0.001
Vitamin B12 0.000005
Calcium Nitrate·4 H2O 0.1
Magnesium Sulfate 0.04884
Potassium Chloride 0.4
Sodium Chloride 6.0
Sodium Phosphate Dibasic 0.8
D-Glucose 2.0
Glutathione, Reduced 0.001
Phenol Red·Na 0.0053

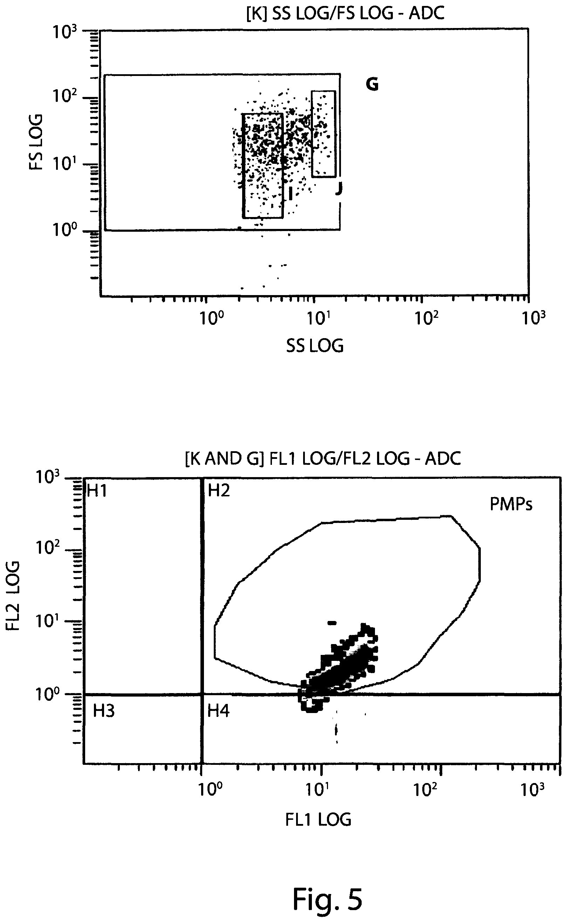

### Detection of platelets and microparticles by flow-cytometry (Figure 1 and 5).

For the detection of microparticles by flow-cytometry, the dimensional gate corresponding to microparticles was set calibrating the instrument by means of Megamix (American Diagnostic), a mixture of microspheres of 3 different sizes (0.5; 0.9 and 3 micron).

The microparticles prepared as indicated in the materials and methods, were marked incubating them for 30 minutes with the fluorescent anti-CD61 antibody (Beckman Coulter), antigen with exclusively platelet localization.

Platelets were analyzed setting the morphological gate with a control without transfection prepared as describe in the materials and methods, and using a fluorescent anti-CD61 antibody (Beckman Coulter)-fitc and detecting the fluorescence emitted from the siRNA TYE 563 DS Transfection Control (IDT).

All the trials were carried out using FC500 flow-cytometer (Beckman Coulter).

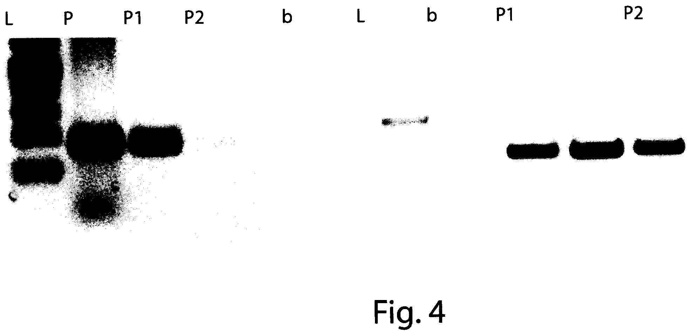

### RT-PCR platelets transfected with siRNA (Fig. 4)

The transfected platelets were prepared as specified in the materials and methods, from point 1 to point 7, using a siRNA directed against HPRT1 at the concentration of 200nM.

The control was obtained washing platelets according to the method previously described and blocking the preparation at point 3 and inserting into the medium 200 nM of siRNA directed against HPRT1.

RT-PCR was carried out as follows: the total RNA was extracted from platelets preparations using TRIzol (Invitrogen) according to the chloroform/isopropanol method of extraction.

The total RNA was reverse-transcribed using iScript (Biorad) according to the manufacturer's instructions.

The PCR was carried out using the following primers: HPRT (for: TGAGGATTTGGAAAGGGTGT (SEQ ID NO: 47) rev: TGTAATCCAGCAGGTCAGCA(SEQ ID NO: 48)); Beta 2 Microglobulin (for: TGACTTTGTCACAGCCCAAGATAG (SEQ ID NO: 49) rev: CTCTAAGTTGCCACCCCTCCTAG(SEQ ID NO: 50)).

### Endothelial cell (HUVEC) detection by flow-cytometry (figure 6)

HUVEC Endothelial cells (Lonza) were cultured in EBM2 Bullet Kit medium (Lonza) into 12-well plates until reaching about 75% of confluence.

Microparticles of platelet origin obtained as previously described after 48 hours of platelet incubation, were resuspended in 1 ml of RPMI 1640 medium and added to the endothelial cell culture.
After 6 hours of co-incubation, the supernatant was removed, endothelial cells were washed for two times in PBS and then removed from platlets by mild trypsinization, resuspended in 1ml of PBS for the flow cytometry analysis.

The morphological gate was set using HUVEC cells of the control not preincubated with microparticles.

The fluorescence emitted from siRNA TYE 563 DS Transfection Control (IDT) was detected.

All the trials were carried out using FC500 flow-cytometer (Beckman Coulter).

### Results

Fluorescent Scrambled-siRNA (Integrated DNA Techonologies) was inserted into human platelets washed (3.5x10⁵) according to the above-described procedure, in order to verify the transfection efficacy.

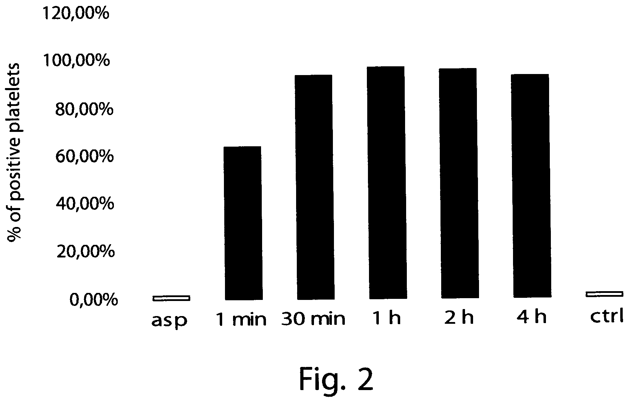

Through flow-citometry tests, a high transfection efficacy was demonstrated (Figure 1). Particuarly, we demonstrated that after 1h of incubation, almost an optimal efficacy percentage of transfection is achieved (about 97% of efficacy) (Figure 2).

A high transfection efficacy (> 95%) is also achieved incubating platelets only for 30 minutes, thus reducing cellular stress induced by transfection.

We carried out tests of platelet mRNA silencing, evaluating mRNA degradation of HPRT1 gene (Hypoxanthine-guanine phosphoribosyltransferase) in platelets washed (3x10⁵) and transfected with 200 nM of siRNA directed against HPRT1 (HPRT-S1 DS Positive Control IDT).

mRNA silencing was verified by semi-quantitative PCR.

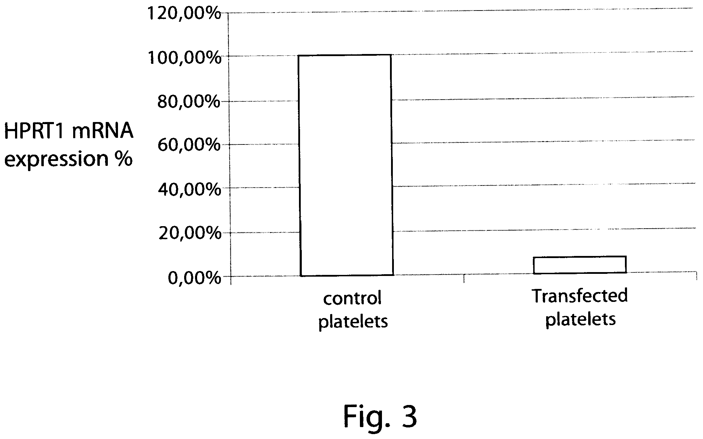

A silencing efficacy of the mRNA of interest equal to 94% was obtained (Figure 3 e 4). Furthermore, washed human platelets were induced to produce microparticles by the method previously described and it was evaluated by flow-citometry whether the fluorescent siRNA, previously transfected into the same platelets, is transferred into microparticles produced (Figure 5). In fact it was demonstrated that 96% of microparticles contains the siRNA previously transfected (Figure 5). Human endothelial cells were incubated in culture (HUVEC) with microparticles deriving from platelets transfected with siRNA verifying that fluorescent siRNA, deriving from platelets from which they were produced, is transferred into about 38% of HUVEC cells (Figure 6).

### EXAMPLE 2: Study on NOD/SCID mouse leukocytes after the intravenous inoculum of human platelets transfected with fluorescent siRNA and evaluation of silencing capability of platelets or microparticles transfected with siRNA

Immunodeficient NOD/SCID mice, therefore unable of immunologically reacting to human platelet infusion, were inoculated by intravenous injection, with 200 µl of a suspension of 5x10⁸ human platelets transfected according to the method described in the example 1 with fluorescent siRNA.

24 hours after the inoculum, mice were sacrificed and a whole blood cardiac sampling was carried out.
Mouse whole blood was co-incubated for 30 minutes with murine antibody anti-CD45 (BD Pharmingen).

The fluorescence emitted from siRNA TYE 563 DS Transfection Control (IDT) in mouse leukocytes marked with antibody anti-CD45 (BD Pharmingen) after 30 minutes of incubation was detected.
All the tests were carried out using flow-cytometer FC500 (Beckman Coulter). The experimental results in vivo, with the experimental animal, which show the persistence in the bloodstream of platelets "transfected" with siRNA and the transfer of the latter to murine leukocytes are shown in Figure 7.

### EXAMPLE 3: GFP detection on human platelets transfected by flow-cytometry (Figure 8).

A total of 1 µg of plasmid containing the sequence of GFP (Pmax, Lonza) was inserted into human platelets using the method described in the example 1, replacing the plasmid with siRNA in the above-described method.

After, platelets were analyzed setting the morphological gate with a platelet suspension of control without transfection prepared as described in the materials and methods, and detecting the fluorescence emitted from GFP produced by Pmax plasmid (max emission 509 nm). All the tests were carried out using the instrument FC500 (Beckman Coulter). Through flow-cytometry it was observed that human platelet transfection with recombinant plasmid containing the sequence for GFP (Green Fluorescent Protein) caused its translation and therefore the expression of plasmid product (Figure 8).

### EXAMPLE 4: Method of platelet or microparticle transfection and evaluation of the transfection percentage

### Materials and methods

### Method of platelet or microparticle transfection

Venous peripheral blood was harvested and collected in tubes containing 3.8% of sodium citrate as anticoagulant (1:9 citrate-blood v/v).
1. The sample was centrifuged at 120 g for 10 minutes in order to obtain PRP (platelet-rich plasma).
2. Proceeding in the isolation of plasma platelets by common methods (platelet washing or gel-filtration).
3. Platelets thus isolated were resuspended in RPMI 1640 added with antibiotics (100 U of Penicillin and 100 U of Streptomycin) or in the same plasma of the donor, at a concentration comprised between 2x10⁵ and 1x10⁶ per microliter, the lower limit of said concentration being 130,000 platelets/microliter, and rates of one milliliter were transferred into a 24-well plate and incubated at 37°C in controlled atmosphere of 5% CO₂.
4. Then, the transfection medium is prepared, inserting 32.4 microliter of absolute ethyl alcohol into a tube, 3 microliters of a polyethylenimmine solution (0.1% in water) and 164.6 microliters of RPMI 1640 medium.
5. After waiting for 5 minutes, the siRNA of interest was added to the above-mentioned mixture to the maximum concentration of 200 nM and after 15 minutes of incubation at room temperature, the solution was transferred into the well in which 1 ml of platelet suspension had previously been placed.
6. After 5 minutes of incubation at 37°C, the transfection was interrupted: by platelet centrifugation (at 3,000 g for 10 minutes in the presence of PGI2 0.2 microM and, then respuspending them in 3 ml of the culture medium RPMI 1640) or by diluting the transfection medium with the culture medium RPMI 1640 until achieving a total amount of 7 milliliters.
7. Within 2 hours after the interruption of the transfection reaction, it is possible to point out (by PCR or rt-PCR) the degradation of the mRNA of interest, a specific target of the siRNA previously inserted, into platelets.

Instead, in order to obtain platelet microparticles containing the siRNA previously transfected into platelets, it is necessary to carry out the previously described operations up to point 5 and then continuing as follows:
6. Incubating platelet suspension at 37°C in controlled atmosphere of 5% CO₂ together with the transfection medium for 24/48 h. Or alternatively stimulating them with 2.5 mM of CaCl₂, 20 µg/ml of Collagen and 1 U/ml of thrombin for 30 minutes at 37°C and blocking the stimulus with 2.5 mM of EDTA.
7. Centrifuging platelets at 3,000 g for 10 minutes.
8. Recovering supernatant and further centrifuging it at 12,000 g for 10 minutes in order to remove possible remained platelets.
9. Centrifuging supernatant at 160,000 g for 1 hour at 4°C.
10. Removing supernatant and resuspending microparticle pellet in 1 milliliter of RPMI 1640 (verifying microparticles concentration using a flow-cytometer).

The composition of RPMI 1640 medium is (grams/liter): the list of components expressed as grams/liter
L-Arginine 0.2
L-Asparagine 0.05
L-Aspartic Acid 0.02
L-Cystine·2HCl 0.0652
L-Glutamic Acid 0.02
L-Glutamine 0.3
Glycine 0.01
L-Histidine 0.015
Hydroxy-L-Proline 0.02
L-Isoleucine 0.05
L-Leucine 0.05
L-Lysine-HCl 0.04
L-Methionine 0.015
L-Phenylalanine 0.015
L-Proline 0.02
L-Serine 0.03
L-Threonine 0.02
L-Tryptophan 0.005
L-Tyrosine·2Na·2H2O 0.02883
L-Valine 0.02
Biotin 0.0002
Choline Chloride 0.003
Folic Acid 0.001
myo-Inositol 0.035
Niacinamide 0.001
D-Pantothenic Acid Hemicalcium 0.00025
PABA 0.001
Pyridoxine·HCl 0.001
Riboflavin 0.0002
Thiamine·HCl 0.001
Vitamin B12 0.000005
Calcium Nitrate·4 H2O 0.1
Magnesium Sulfate 0.04884
Potassium Chloride 0.4
Sodium Chloride 6.0
Sodium Phosphate Dibasic 0.8
D-Glucose 2.0
Glutathione, Reduced 0.001
Phenol Red·Na 0.0053

### Platelet detection by flow-cytometry (Figure 9).

Platelets were analyzed setting the morphological gate with a control without transfection prepared as describe in the materials and methods of example 4, using a fluorescent anti-CD61 antibody (Beckman Coulter)-fitc and detecting the fluorescence emitted from the siRNA TYE 563 DS Transfection Control (IDT).

All the trials were carried out using FC500 flow-cytometer (Beckman Coulter).

### EXAMPLE 5: Method of platelet or microparticle transfection and evaluation of the transfection percentage

### Materials and methods

### Method of platelet or microparticle transfection

Venous peripheral blood was harvested and collected in tubes containing 3.8% of sodium citrate as anticoagulant (1:9 citrate-blood v/v).
1. The sample was centrifuged at 120 g for 10 minutes in order to obtain PRP (platelet-rich plasma).
2. Proceeding to the isolation of plasma platelets by common methods (platelet washing or gel-filtration).
3. Platelets thus isolated were resuspended in RPMI 1640 added with antibiotics (100 U of Penicillin and 100 U of Streptomycin) or in the same plasma of the donor, at a concentration comprised between 2x10⁵ and 1x10⁶ per microliter, the lower limit of said concentration being 130,000 platelets/microliter, and rates of one milliliter were transferred to a 24-well plate and incubated at 37°C in controlled atmosphere of 5% CO₂.
4. Then, the transfection medium is prepared, inserting 32.4 microliter of absolute ethyl alcohol, 120 microliters of a polylysine solution (0.1% in water) and 47.6 microliters of RPMI 1640 medium.
5. After waiting for 5 minutes, the siRNA of interest was added to the above-mentioned mixture of the siRNA of interest to the maximum concentration of 200 nM and after 15 minutes of incubation at room temperature, the solution was transferred into the well in which 1 ml of platelet suspension had previously been placed.
6. After 5 minutes of incubation at 37°C, the transfection was interrupted: by platelet centrifugation (at 3,000 g for 10 minutes in the presence of PGI2 0.2 microM and, then respuspending them in 3 ml of the culture medium RPMI 1640) or by diluting the transfection medium with the culture medium RPMI 1640 until achieving a total amount of 7 milliliters.
7. Within 2 hours after the interruption of the transfection reaction, it is possible to point out (by PCR or rt-PCR) the degradation of the mRNA of interest, a specific target of the siRNA previously inserted, into platelets.

Instead, in order to obtain platelet microparticles containing the siRNA previously transfected into platelets, it is necessary to carry out the previously described operations up to point 5 and then continuing as follows:
11. Incubating platelet suspension at 37°C in controlled atmosphere of 5% CO₂ together with the transfection medium for 24/48 h. Or alternatively stimulating them with 2.5 mM of CaCl₂ , 20 µg/ml of Collagen and 1 U/ml of thrombin for 30 minutes at 37°C and blocking the stimulus with 2.5 mM of EDTA.
12. Centrifuging platelets at 3,000 g for 10 minutes.
13. Recovering supernatant and further centrifuging it at 12,000 g for 10 minutes in order to remove possible remained platelets.
14. Centrifuging supernatant at 160,000 g for 1 hour at 4°C.
15. Removing supernatant and resuspending microparticle pellet in 1 milliliter of RPMI 1640 (verifying microparticles concentration using a flow-cytometer).

The composition of RPMI 1640 medium is (grams/liter): the list of components expressed as grams/liter
L-Arginine 0.2
L-Asparagine 0.05
L-Aspartic Acid 0.02
L-Cystine·2HCl 0.0652
L-Glutamic Acid 0.02
L-Glutamine 0.3
Glycine 0.01
L-Histidine 0.015
Hydroxy-L-Proline 0.02
L-Isoleucine 0.05
L-Leucine 0.05
L-Lysine-HCl 0.04
L-Methionine 0.015
L-Phenylalanine 0.015
L-Proline 0.02
L-Serine 0.03
L-Threonine 0.02
L-Tryptophan 0.005
L-Tyrosine·2Na·2H2O 0.02883
L-Valine 0.02
Biotin 0.0002
Choline Chloride 0.003
Folic Acid 0.001
myo-Inositol 0.035
Niacinamide 0.001
D-Pantothenic Acid Hemicalcium 0.00025
PABA 0.001
Pyridoxine·HCl 0.001
Riboflavin 0.0002
Thiamine·HCl 0.001
Vitamin B12 0.000005
Calcium Nitrate·4 H2O 0.1
Magnesium Sulfate 0.04884
Potassium Chloride 0.4
Sodium Chloride 6.0
Sodium Phosphate Dibasic 0.8
D-Glucose 2.0
Glutathione, Reduced 0.001
Phenol Red·Na 0.0053

### Platelet detection by flow-cytometry (Figure 10).

Platelets were analyzed setting the morphological gate with a control withouth transfection prepared as describe in the materials and methods of example 5, using a fluorescent anti-CD61 antibody (Beckman Coulter)-fitc and detecting the fluorescence emitted from the siRNA TYE 563 DS Transfection Control (IDT).
All the trials were carried out using FC500 flow-cytometer (Beckman Coulter).

## Claims

1. Method for transfecting platelets isolated from peripheral blood with exogenous genetic material, said method comprising or consisting of the following steps:
a) adding a mixture comprising or consisting of genetic material and a transfection medium, said transfection medium comprising ethyl alcohol and at least one polyamine chosen from the group consisting of polyethylenimmine and polylysine, to a suspension of isolated platelets isolated from peripheral blood suspended in a suitable suspension medium; and
b) incubating until obtaining a percentage of transfected platelets isolated from peripheral blood equal at least to 20%, preferably from 50 to 100%, even more preferably from 80 to 100%, of the entire platelet population, interrupting the transfection and isolating the platelets.

2. Method for the preparation of platelet microparticles deriving from platelets isolated from peripheral blood transfected with exogenous genetic material, said method comprising or consisting of the following steps: a) adding a mixture comprising or consisting of genetic material and a transfection medium, said transfection medium comprising ethyl alcohol and at least one polyamine chosen from the group consisting of polyethylenimmine and polylysine, to a suspension of isolated mature platelets suspendend in a suitable medium of suspension; and b) incubating, possibly stimulating platelet activation, until obtaining microparticles, interrupting the transfection and isolating microparticles.

3. Method according to any one of claims 1-2, wherein the concentration of platelets isolated from peripheral blood in suspension of step a) ranges from 20,000 platelets per microliter to 2 millions, preferably from 200,000 to 1,000,000 per microliter, even more preferably 250,000-350,000, even more preferably 300,000 per microliter.

4. Method according to any one of claims 1-3, wherein the genetic material is selected from the group consisting of siRNA, shRNA, ceRNA, DNA, plasmids.

5. Population of platelets isolated from peripheral blood and/or a population of platelet microparticles obtained from said population of platelets, which platelets have been transfected with exogenous genetic material, and wherein from 50% to 100%, even more preferably from 80% to 100% of the platelets are transfected platelets; and wherein said population of platelets and said population of platelets microparticles are obtainable by the method as defined in any one of claims 1-4.

6. Population of platelets isolated from peripheral blood and/or the population of platelet microparticles obtained from said population of platelets according to claim 5, wherein said genetic material is selected from the group consisting of siRNA, shRNA, ceRNA, DNA, plasmids.

7. Population of platelets isolated from peripheral blood and/or the population of platelet microparticles according to any one of claims 5-6, for use in therapy as vector in the transportation and transfection of genetic material to acceptor cells.

8. Population of platelets isolated from peripheral blood and/or the population of platelet microparticles according to any one of claims 5-6, for use in gene therapy or cell therapy.

9. Population of platelets isolated from peripheral blood and/or the population of platelet microparticles according to claim 8, for the use according to claim 8, wherein the target gene of the gene therapy is a gene of the same platelets or of the acceptor cells.

10. Population of platelets isolated from peripheral blood and/or the population of platelet microparticles according to claim 8, for the use according to any one of claims 8-9, wherein when said genetic material is selected from the group consisting of siRNA, shRNA, or in said siRNA and shRNA contained into plasmids, the gene therapy consists of post-transcriptional silencing of a target gene.

## Patentansprüche

1. Verfahren zur Transfektion von aus peripherem Blut isolierten Thrombozyten mit exogenem genetischem Material, wobei das Verfahren die folgenden Schritte umfasst oder daraus besteht:
a) Zugabe einer Mischung, die genetisches Material und ein Transfektionsmedium umfasst oder daraus besteht, wobei das Transfektionsmedium Ethylalkohol und mindestens ein Polyamin, ausgewählt aus der Gruppe, bestehend aus Polyethylenimin und Polylysin, umfasst, zu einer Suspension von isolierten Thrombozyten, die aus peripherem Blut isoliert wurden und in einem geeigneten Suspensionsmedium suspendiert sind; und
b) Inkubation bis zum Erhalt eines Prozentsatzes von transfizierten, aus peripherem Blut isolierten Thrombozyten von mindestens 20%, vorzugsweise von 50 bis 100%, noch bevorzugter von 80 bis 100%, der gesamten Thrombozytenpopulation, Unterbrechung der Transfektion und Isolierung der Thrombozyten.

2. Verfahren zur Herstellung von Thrombozyten-Mikropartikeln, die aus Thrombozyten stammen, die aus peripherem Blut isoliert und mit exogenem genetischem Material transfiziert wurden, wobei das Verfahren die folgenden Schritte umfasst oder daraus besteht: a) Zugabe einer Mischung, die genetisches Material und ein Transfektionsmedium umfasst oder daraus besteht, wobei das Transfektionsmedium Ethylalkohol und mindestens ein Polyamin, ausgewählt aus der Gruppe, bestehend aus Polyethylenimin und Polylysin, umfasst, zu einer Suspension von isolierten reifen Thrombozyten, die in einem geeigneten Suspensionsmedium suspendiert sind; und b) Inkubation, gegebenenfalls Stimulierung der Thrombozytenaktivierung, bis zum Erhalt von Mikropartikeln, Unterbrechung der Transfektion und Isolierung der Mikropartikel.

3. Verfahren nach einem der Ansprüche 1-2, wobei die Konzentration der aus peripherem Blut isolierten Thrombozyten in der Suspension von Schritt a) im Bereich von 20.000 Thrombozyten pro Mikroliter bis 2 Millionen, vorzugsweise von 200.000 bis 1.000.000 pro Mikroliter, noch bevorzugter 250.000-350.000, noch bevorzugter 300.000 pro Mikroliter liegt.

4. Verfahren nach einem der Ansprüche 1-3, wobei das genetische Material ausgewählt ist aus der Gruppe, bestehend aus siRNA, shRNA, ceRNA, DNA, Plasmiden.

5. Population von Thrombozyten, die aus peripherem Blut isoliert wurden, und/oder Population von Thrombozyten-Mikropartikeln, die aus der Population von Thrombozyten erhalten wurden, wobei die Thrombozyten mit exogenem genetischem Material transfiziert wurden, und wobei 50% bis 100%, noch bevorzugter 80% bis 100% der Thrombozyten transfizierte Thrombozyten sind; und wobei die Population von Thrombozyten und die Population von Thrombozyten-Mikropartikeln durch das Verfahren nach einem der Ansprüche 1-4 erhältlich sind.

6. Population von Thrombozyten, die aus peripherem Blut isoliert wurden, und/oder Population von Thrombozyten-Mikropartikeln, die aus der Population von Thrombozyten erhalten wurde, nach Anspruch 5, wobei das genetische Material ausgewählt ist aus der Gruppe, bestehend aus siRNA, shRNA, ceRNA, DNA, Plasmiden.

7. Population von Thrombozyten, die aus peripherem Blut isoliert wurden, und/oder Population von Thrombozyten-Mikropartikeln nach einem der Ansprüche 5-6 zur Verwendung in der Therapie als Vektor beim Transport und der Transfektion von genetischem Material zu/in Akzeptorzellen.

8. Population von Thrombozyten, die aus peripherem Blut isoliert wurden, und/oder Population von Thrombozyten-Mikropartikeln nach einem der Ansprüche 5-6 zur Verwendung in der Gen- oder Zelltherapie.

9. Population von Thrombozyten, die aus peripherem Blut isoliert wurden, und/oder Population von Thrombozyten-Mikropartikeln nach Anspruch 8, zur Verwendung nach Anspruch 8, wobei das Zielgen der Gentherapie ein Gen der gleichen Thrombozyten oder der Akzeptorzellen ist.

10. Population von Thrombozyten, die aus peripherem Blut isoliert wurden, und/oder Population von Thrombozyten-Mikropartikeln nach Anspruch 8, zur Verwendung nach einem der Ansprüche 8-9, wobei, wenn das genetische Material ausgewählt ist aus der Gruppe, bestehend aus siRNA, shRNA, oder in der siRNA und shRNA in Plasmiden enthalten ist, die Gentherapie aus posttranskriptionalem Silencing eines Zielgens besteht.

## Revendications

1. Procédé de transfection de plaquettes isolées du sang périphérique avec du matériau génétique exogène, ledit procédé comprenant ou constitué des étapes suivantes :
a) d'addition d'un mélange comprenant ou constitué de matériau génétique et d'un milieu de transfection, ledit milieu de transfection comprenant de l'alcool d'éthyle et au moins une polyamine choisie dans le groupe constitué de la polyéthylenimine et de la polylysine, à une suspension de plaquettes isolées qui sont isolées du sang périphérique en suspension dans un milieu de suspension convenable ; et
b) d'incubation jusqu'à l'obtention d'un pourcentage de plaquettes transfectées isolées du sang périphérique égal à au moins 20 %, préférablement de 50 à 100 %, même plus préférablement de 80 à 100 %, de la population entière des plaquettes, en interrompant la transfection et en isolant les plaquettes.

2. Procédé de préparation de microparticules plaquettaires provenant de plaquettes isolées du sang périphérique transfectées avec du matériau génétique exogène, ledit procédé comprenant ou étant constitué des étapes suivantes :
a) d'addition d'un mélange comprenant ou constitué de matériau génétique et d'un milieu de transfection, ledit milieu de transfection comprenant de l'alcool d'éthyle et au moins une polyamine choisie dans le groupe constitué de la polyéthylenimine et de la polylysine, à une suspension de plaquettes matures isolées en suspension dans un milieu de suspension convenable ; et
b) d'incubation, de manière possible en stimulant l'activation des plaquettes, jusqu'à l'obtention de microparticules, en interrompant la transfection et en isolant les microparticules.

3. Procédé selon l'une quelconque des revendications 1 à 2, la concentration des plaquettes isolées du sang périphérique dans la suspension de l'étape a) s'étend de 20 000 plaquettes par microlitre à 2 millions, préférablement de 200 000 à 1 000 000 par microlitre, même plus préférablement de 250 000 à 350 000, même plus préférablement de 300 000 par microlitre.

4. Procédé selon l'une quelconque des revendications 1 à 3, le matériau génétique étant sélectionné dans le groupe constitué de l'ARNsi, ARNsh, ARNce, de l'ADN, des plasmides.

5. Population de plaquettes isolées du sang périphérique et/ou population de microparticules plaquettaires obtenues à partir de ladite population de plaquettes, lesquelles plaquettes ont été transfectées avec du matériau génétique exogène, et de 50 % à 100 %, même plus préférablement de 80 % à 100 % des plaquettes étant des plaquettes transfectées ; et ladite population de plaquettes et ladite population de microparticules plaquettaires pouvant être obtenues par le procédé tel que défini selon l'une quelconque des revendications 1 à 4.

6. Population de plaquettes isolées du sang périphérique et/ou population de microparticules plaquettaires obtenues à partir de ladite population de plaquettes selon la revendication 5, ledit matériau génétique étant sélectionné à partir du groupe constitué de l'ARNsi, ARNsh, ARNce, de l'ADN, des plasmides.

7. Population de plaquettes isolées du sang périphérique et/ou population de microparticules plaquettaires selon l'une quelconque des revendications 5 à 6, pour l'utilisation en thérapie comme vecteur dans le transport et la transfection de matériau génétique à des cellules acceptrices.

8. Population de plaquettes isolées du sang périphérique et/ou population de microparticules plaquettaires selon l'une quelconque des revendications 5 à 6, pour l'utilisation en thérapie génique ou en thérapie cellulaire.

9. Population de plaquettes isolées du sang périphérique et/ou population de microparticules plaquettaires selon la revendication 8, pour l'utilisation selon la revendication 8, le gène cible de la thérapie génique étant un gène des mêmes plaquettes ou des cellules acceptrices.

10. Population de plaquettes isolées du sang périphérique et/ou population de microparticules plaquettaires selon la revendication 8, pour l'utilisation selon l'une quelconque des revendications 8 à 9, lorsque ledit matériau génétique est sélectionné dans le groupe constitué de l'ARNsi, ARNsh, ou dans ledit ARNsi et ARNsh contenus dans des plasmides, la thérapie génique consiste en silençage post-transcriptionnel d'un gène cible.
